(19) **Europäisches Patentamt / European Patent Office / Office européen des brevets**

(11) **EP 2 001 437 B1**

(12) **FASCICULE DE BREVET EUROPEEN**

(45) Date de publication et mention de la délivrance du brevet:
**22.02.2017 Bulletin 2017/08**

(21) Numéro de dépôt: **07731757.6**

(22) Date de dépôt: **16.03.2007**

(51) Int Cl.:
*A61K 8/60* *(2006.01)*  *A61Q 19/10* *(2006.01)*
*A61Q 5/02* *(2006.01)*  *A61K 8/46* *(2006.01)*

(86) Numéro de dépôt international:
**PCT/FR2007/050941**

(87) Numéro de publication internationale:
**WO 2007/110526 (04.10.2007 Gazette 2007/40)**

(54) **NOUVEAU PROCÉDÉ D'AMÉLIORATION DE LA TOLÉRANCE OCULAIRE DE COMPOSITIONS MOUSSANTES ET/OU DÉTERGENTES À USAGE CUTANÉ**

NEUES VERFAHREN ZUR VERBESSERUNG DER OKULARTOLERANZ VON SCHÄUMENDEN UND TENSIDE ENTHALTENDEN ZUSAMMENSETZUNGEN ZUR TOPISCHEN HAUTAPPLIKATION

NEW METHOD OF IMPROVING THE OCULAR TOLERANCE OF FOAMING AND/OR DETERGENT COMPOSITIONS FOR SKIN USE

(84) Etats contractants désignés:
**AT BE BG CH CY CZ DE DK EE ES FI FR GB GR HU IE IS IT LI LT LU LV MC MT NL PL PT RO SE SI SK TR**
Etats d'extension désignés:
**AL BA HR MK RS**

(30) Priorité: **24.03.2006 FR 0651019**

(43) Date de publication de la demande:
**17.12.2008 Bulletin 2008/51**

(73) Titulaire: **Société d'Exploitation de Produits pour les Industries Chimiques SEPPIC**
**75007 Paris (FR)**

(72) Inventeurs:
• **ROSO, Alicia**
  **F-81710 Saix (FR)**
• **AMALRIC, Chantal**
  **F-81700 Blan (FR)**
• **TABACCHI, Guy**
  **F-75012 Paris (FR)**

(74) Mandataire: **Conan, Philippe Claude**
**L'Air Liquide SA**
**Direction de la Propriété Intellectuelle**
**75, quai d'Orsay**
**75321 Paris Cedex 07 (FR)**

(56) Documents cités:
**WO-A-86/01512**  **WO-A-03/094864**
**DE-A1- 10 044 662**  **US-A1- 2002 123 438**

**Description**

La présente invention a pour objet un nouveau procédé pour améliorer la tolérance oculaire des compositions à usage topique, et notamment les compositions moussantes et/ou détergentes.

L'invention trouve application préférentiellement dans le domaine cosmétique et dermocosmétique, dans le domaine dermopharmaceutique et pharmaceutique, mais également dans le domaine de l'industrie textile par exemple pour le traitement de fibres textiles synthétiques ou naturelles tissées ou tricotées ou encore dans le domaine de l'industrie papetière par exemple pour la fabrication de papier à usage sanitaire ou domestique.

La mise au point de formules nettoyantes pour le visage et pour les cheveux, présentées sous forme de shampoings, de lotions, requièrent une bonne tolérance oculaire. Cette préoccupation est particulièrement importante dans le développement de formules nettoyantes pour les bébés et les enfants, mais elle constitue aussi une exigence pour les utilisateurs adultes de ces produits. D'autre part, la recherche d'une bonne tolérance oculaire est également étendue à l'ensemble des produits lavant pour le corps, les gels douche et les bains moussants.

D'autre part, la génération de mousse par ces formulations lors de leur utilisation constitue un élément clé des performances requises car le consommateur la considère comme un indicateur critique de l'efficacité du nettoyage.

Plusieurs catégories de tensioactifs sont utilisées pour la préparation de formulations à visée nettoyante : des tensioactifs cationiques, anioniques, amphotères ou non ioniques.

Les tensioactifs anioniques, comme les tensioactifs anioniques sulfatés, constituent une classe de tensioactifs fréquemment utilisée en raison de leurs bonnes propriétés moussantes. Cependant, ces tensioactifs induisent des réactions d'intolérance et/ou d'irritation, notamment oculaire et/ou cutanée.

Pour diminuer l'ampleur de ces phénomènes, et sans toutefois les éliminer complètement, on préfère plutôt utiliser les alkyléthersulfates que les alkylsulfates.. Une autre solution elle-aussi partiellement satisfaisante, consiste à associer ces tensioactifs sulfatés à des tensioactifs amphotères, tels que les bétaïnes, comme l'AMONYL™ 380BA et l'AMONYL™ 675SB commercialisés par la société SEPPIC à des tensioactifs cationiques tels que le « cocoamidopropyl bétaïnamide MEA chloride » commercialisé sous l'appellation MONTALINE™ C40 par la société SEPPIC ou le cétyltrimonium ammonium chloride commercialisé sous l'appellation DEHYQUART™ ACA par COGNIS ou encore à des acylats de protéines ou les dérivés acylés d'acides aminés, tels que le triéthanolamine cocoyl glutamate commercialisé sou l'appellation ACYLGLUTAMATE™ C12S par la société AJINOMOTO. La plupart de ces associations se révèlent cependant peu efficaces pour diminuer l'irritation oculaire et. ce problème subsiste, quand bien même on supprime totalement les tensioactifs anioniques pour les remplacer par ces tensioactifs amphotères ou cationiques.

Une autre solution pour améliorer la tolérance oculaire des formulations nettoyantes, consiste à associer les tensioactifs sulfatés à des tensioactifs de type non ioniques éthoxylés. On a essayé par exemple des associations avec des esters de sorbitan éthoxylés, tels que le Polysorbate 20 ou le Polysorbate 60, commercialisés par la société SEPPIC sous les appellations respectives de Montanox™ 20 et de Montanox™ 60, et tel que le PEG-80 Sorbitan laurate, commercialisé sous l'appellation Montanox™ MLS 80 par la société SEPPIC, ou des associations avec des esters de glycérol éthoxylés comme celle associant le PEG-40 glycéryl cocoate et le Sodium coceth sulfate dans la composition commercialisée par la société SEPPIC sous l'appellation ORONAL™ LCG. De telles associations, si elles permettent de réduire l'irritation oculaire de façon efficace lorsque la quantité de co-tensioactifs non ioniques éthoxylés est utilisée dans des proportions élevées et représentent au moins 40% de la matière sèche du mélange alkylethersulfate/co-tensioactif non ionique éthoxylé, ont cependant pour conséquences de diminuer de façon très significative le volume de mousse formée lors de l'utilisation par le consommateur et dans certains cas, d'altérer la stabilité de la mousse dans le temps.

La demande internationale publiée sous le numéro WO 03/094864 décrit un gel douche contenant du lauryl éthersulfate de sodium et un mélange de xilitol et de xylityl-glucoside.

La demande de brevet des Etats Unis d'Amérique publiée sous le numéro US 2002/0123438 A1 divulgue une composition détergente peu irritante pour les yeux comprenant un copolymère acrylique avec un chaîne hydrophobe, un polyalcoxy ester de polyol et un tensioactif anionique.

Les inventeurs ont donc cherché à développer une nouvelle solution pour améliorer la tolérance oculaire des compositions cosmétiques, dermocosmétiques, dermopharmaceutiques ou pharmaceutiques.

C'est pourquoi selon un premier aspect, l'invention a pour objet un procédé pour améliorer la tolérance oculaire d'une composition à usage topique, caractérisé en ce que l'on incorpore dans ladite composition, une quantité efficace d'un composé de formule (I):

$$R_1\text{-}O\text{-}(G)_x\text{-}H \qquad (I)$$

dans laquelle :

x représente un nombre décimal compris entre 1 et 5,

G représente le reste d'un sucre réducteur et
R₁ représente un radical monovalent de formule (A) :

$$-CH_2-(CHOH)_n-CH_2-OH \qquad (A)$$

dans laquelle n est un nombre entier égal à 2, 3 ou 4, ou bien
R₁ représente un radical monovalent de formule (B) :

$$-(CH_2-CHOH-CH_2-O)_m-H \qquad (B)$$

dans laquelle m est un nombre entier égal à 1, 2 ou 3,
ou d'un mélange de composés de formules (I).

Par quantité efficace, on désigne, dans la définition du procédé tel que défini ci-dessus, une quantité telle, que la composition finale, obtenue par ledit procédé, ait un indice HETCAM inférieur à 5 et de préférence inférieur ou égal à 3. La méthode de détermination d'un tel indice est décrite au paragraphe B de la partie expérimentale du présent exposé. Selon un mode particulier du procédé tel que défini ci-dessus, par quantité efficace de composé de formule (I), on désigne une proportion massique de 0,5% à 10% de la composition finale et tout particulièrement de 1% à 5%.

L'expression "à usage topique" utilisée dans la définition du procédé tel que défini ci-dessus, signifie que ladite composition est mise en oeuvre par application sur la peau, les cheveux, le cuir chevelu ou les muqueuses, qu'il s'agisse d'une application directe dans le cas d'une composition cosmétique, dermocosmétique, dermopharmaceutique ou pharmaceutique ou d'une application indirecte par exemple dans le cas d'un produit d'hygiène corporelle sous forme de lingette en textile ou en papier ou de produits sanitaires destinés à être en contact. avec la peau ou les muqueuses

Par sucre réducteur, on désigne, dans la définition de la formule (I) du composé mis en en oeuvre dans le procédé tel que défini ci-dessus, , les dérivés saccharidiques qui ne présentent pas dans leurs structures de liaison glycosidique établie entre un carbone anomérique et l'oxygène d'un groupement acétal tels qu'ils sont définis dans l'ouvrage de référence : Biochemistry », Daniel Voet/Judith G. Voet, p. 250, John Wyley & Sons, 1990). La structure oligomérique (G)ₓ, peut se présenter sous toute forme d'isomérie, qu'il s'agisse d'isomérie optique, d'isomérie géométrique ou d'isomérie de position ; elle peut aussi représenter un mélange d'isomères. Dans la formule (I) telle que définie ci-dessus, le groupe R₁-O- est lié à G par le carbone anomérique du reste saccharide, de manière à former une fonction acétal.

Selon un autre aspect particulier du procédé tel que défini ci-dessus, dans la formule (I), G représente le reste d'un sucre réducteur choisi parmi le glucose, le dextrose, le saccharose, le fructose, l'idose, le gulose, le galactose, le maltose, l'isomaltose, le maltotriose, le lactose, le cellobiose, le mannose, le ribose, le xylose, l'arabinose, le lyxose, l'allose, l'altrose, le dextrane ou le tallose.

Selon un autre aspect particulier du procédé tel que défini ci-dessus, dans la formule (I), G représente le reste d'un sucre réducteur choisi parmi le glucose, le xylose ou l'arabinose.

Selon un aspect particulier de la présente invention, celle-ci a pour objet un procédé tel que défini précédemment, dans lequel la composition à usage topique comporte au moins un tensioactif moussant et/ou détergent.

Par tensioactif moussant et/ou détergent, on désigne les tensioactifs anioniques, cationiques, amphotères ou non ioniques topiquement acceptables habituellement utilisés dans ce domaine d'activité.

Parmi les tensioactifs anioniques que l'on peut associer à ces composés et à ces concentrés, on citera particulièrement les sels de métaux alcalins, les sels de métaux alcalino-terreux, les sels d'ammonium, les sels d'amines, les sels d'aminoalcools des composés suivants : les alkyléthers sulfates, les alkylsulfates, les alkylamidoéthersulfates, les alkylarylpolyéthersulfates, les monoglycérides sulfates, les alpha-oléfinesulfonates, les paraffines sulfonates, les alkylphosphates, les alkylétherphosphates, les alkylsulfonates, les alkylamidesulfonates, les alkylarylsulfonates, les alkylcarboxylates, les alkylsulfosuccinates, les alkyléthersulfosuccinates, les alkylamidesulfosuccinates, les alkylsulfoacétates, les alkylsarcosinates, les acyliséthionates, les N-acyltaurates, les acyllactylates. Parmi les tensioactifs anioniques, on citera également les lipoaminoacides, les lipoprotéines, les lipopeptides, les dérivés des lipoprotéines, les dérivés de protéines, les sels d'acides gras, les sels d'acides d'huile de coprah éventuellement hydrogénée.

Parmi les tensioactifs amphotères que l'on peut associer à ces composés et à ces concentrés, on citera particulièrement les alkylbétaines, les alkylamidobétaines, les sultaines, les alkylamidoalkylsulfobétaines, les dérivés d'imidazolines, les phosphobétaïnes, les amphopolyacétates et les amphopropionates.

Parmi les tensioactifs cationiques que l'on peut associer à ces composés et à ces concentrés, on citera particulièrement les dérivés d'ammoniums quaternaires.

Parmi les tensioactifs non ioniques que l'on peut associer à ces composés et à ces concentrés, on citera particulièrement les alkylpolyglycosides, les dérivés d'huile de ricin, les polysorbates, les amides de coprah, les N-alkylamines, les oxydes d'amines. Parmi les tensioactifs moussants et/ou détergents cités ci-dessus, qui sont des tensioactifs anioniques, il y a plus particulièrement les composés de formule (II) :

$$[R_2\text{-}O\text{-}(CH_2\text{-}CH_2\text{-}O)_p\text{-}SO_3]_rX \qquad (II)$$

dans laquelle:

$R_2$ représente un radical hydrocarboné aliphatique, saturé ou insaturé, linéaire ou ramifié, comportant de 6 à 22 atomes de carbone,
p représente un nombre décimal compris entre 1 et 10, de préférence entre 2 et 4,
r est un nombre entier égal à 1 ou à 2 et
X représente le cation d'un métal alcalin ou d'un métal alcalino-terreux, l'ion ammonium, l'ion hydroxyéthyl ammonium, l'ion tri(hydroxyéthyl) ammonium ou un mélange de composé de formule (II).

Dans la formule (II) telle que définie ci-dessus, X représente par exemple le sodium, le magnésium ou le l'ion ammonium.

Selon un autre aspect particulier du procédé tel que défini précédemment, le rapport massique composés de formule (I) sur agents tensioactifs moussants et/ou détergents présents dans ladite composition à usage topique, est compris entre 1/10 et 10/1 plus particulièrement entre 1/10 et 1/1.

Comme exemple de concentré que l'on peut incorporer dans la composition à usage topique il y a un concentré comprenant pour 100% de sa masse :

- de 8% à 90% massique d'un composé de formule (I) :

$$R_1\text{-}O\text{-}(G)_x\text{-}H \qquad (I)$$

dans laquelle :

x représente un nombre décimal compris entre 1 et 5,
G représente le reste d'un sucre réducteur et
$R_1$ représente un radical monovalent de formule (A) :

$$-CH_2\text{-}(CHOH)_n\text{-}CH_2\text{-}OH \qquad (A)$$

dans laquelle n est un nombre entier égal à 2, 3 ou 4, ou bien
$R_1$ représente un radical monovalent de formule (B) :

$$-(CH_2\text{-}CHOH\text{-}CH_2\text{-}O)_m\text{-}H \qquad (B)$$

dans laquelle m est un nombre entier égal à 1, 2 ou 3,

ou d'un mélange de composés de formules (I) ;
- de 2% à 99% massique d'un composé de formule (II)

$$[R_2\text{-}O\text{-}(CH_2\text{-}CH_2\text{-}O)_p\text{-}SO_3]_rX \qquad (II)$$

dans laquelle:

$R_2$ représente un radical hydrocarboné aliphatique, saturé ou insaturé, linéaire ou ramifié, comportant de 6 à 22 atomes de carbone,
p représente un nombre décimal compris entre 1 et 10, de préférence entre 2 et 4,
r est un nombre entier égal à 1 ou à 2 et
X représente le cation d'un métal alcalin ou d'un métal alcalino-terreux, l'ion ammonium, l'ion hydroxyéthyl ammonium, l'ion tri(hydroxyéthyl) ammonium

ou d'un mélange de composé de formule (II) ; et
- de 0% à 80 % massique d'un solvant topiquement acceptable.

Par solvant topiquement acceptable, on désigne dans le cadre de la présente invention les solvants connus de l'homme du métier pouvant être appliqués sur la peau humaine et/ou animale, sur le cuir chevelu et sur les muqueuses.Le solvant topiquement acceptable est notamment choisi parmi un ou plusieurs éléments d'un groupe constitué par l'eau, des glycols, des polyols, des alcools, des polyols alcoxylés, des éthers de glycols et, plusparticulièrement parmi un ou plusieurs éléments d'un groupe constitué par l'eau, l'éthanol, l'isopropanol, le butylène glycol, l'hexylène glycol, le caprylyl glycol ou 1,2 octanediol, le pentylene glycol ou 1,2 pentanediol, l'Ethylhexylglycerin ou octoxyglycerin, le glycérol, le diglycérol, le triglycérol, l'érythritol, le xylitol, le sorbitol, le butyldiglycol, les polyéthylènes glycols dont le poids moléculaire est compris entre 200 g.mol$^{-1}$ et 8000 g.mol$^{-1}$, le monopropylene glycol, le dipropylene glycol, le butyldiglycol, le methyl-2,-propanediol-1,3.

Avantageusement, le solvant topiquement acceptable précité est choisi parmi l'eau, et parmi un ou plusieurs éléments du groupe des polyols constitué par le xylitol, l'érythritol, le sorbitol, le glycérol et le diglycérol.

Les composés de formule (I) ou les mélanges de composés de formule (I), les composés de formule (II) et le solvant topiquement acceptable peuvent être incorporés dans la composition cosmétique, à usage topique de façon séparée ou bien sous la forme d'un concentré tel que défini ci-dessus. D'autre part, selon une ou plusieurs voies de préparation des composés de formule (I) ou des mélanges de composés de formule (I), qui consiste à faire réagir un sucre réducteur G avec un polyol de formule (AI) :

$$HO\text{-} CH_2\text{-}(CHOH)_n\text{-}CH_2\text{-}OH \qquad (A1)$$

dans laquelle n est un nombre entier égal à 2, 3 ou 4 et/ou de formule (B1),

$$HO\text{-}(CH_2\text{-}CHOH\text{-}CH_2\text{-}O)_m\text{-}H \qquad (B1)$$

dans laquelle m est un nombre entier égal à 1, 2 ou 3,
la quantité de polyol n'ayant pas réagi, lorsque celui-ci a été sélectionné parmi un ou plusieurs éléments du groupe défini précédemment, peut constituer toute ou partie du solvant topiquement acceptable. Dans ce cas, les composés de formule (I) ou les mélanges de composés de formule (I) et le solvant topiquement acceptable sont incorporés dans la composition cosmétique, à usage topique de façon concomitante et les composés de formule (II) peuvent être incorporés dans une étape ultérieure.

Selon un mode particulier du concentré tel que défini précédemment, dans la formule (I), G représente le reste d'un sucre réducteur choisi parmi le glucose, le xylose et l'arabinose.

Selon un autre mode particulier du concentré tel que défini précédemment, dans la formule (II) $R_2$ représente un radical hydrocarboné aliphatique saturé comportant de 8 à 18 atomes de carbone.

Selon un autre mode particulier du concentré tel que défini précédemment, il comprend pour 100% de sa masse :

de 8% à 50% massique d'un composé de formule (I) ou d'un mélange de composés de formule (I),
de 10% à 95% massique d'un composé de formule (II) ou d'un mélange de composés de formule (II) et
de 0 à 80% massique d'un solvant topiquement acceptable.

Le concentré objet de l'invention peut être obtenu par diverses voies.

Une première voie de synthèse consiste en une première étape (a), à introduire un composé de formule (I) ou d'un mélange de composés de formule (I) et un composé de formule (II) ou un mélange de composés de formule (II) dans un réacteur selon un rapport massique maîtrisé, et à soumettre ce mélange à une agitation mécanique efficace, dans des conditions de température permettant d'assurer l'homogénéité du mélange, préférentiellement entre 20°C et 90°C.

Si nécessaire, une seconde étape (b) consiste à introduire un solvant topiquement acceptable tel que défini précédemment au mélange obtenu lors de l'étape (a), et à poursuivre l'agitation jusqu'à l'obtention d'un concentré homogène.

Une deuxième voie de synthèse du concentré selon l'invention consiste à synthétiser lors d'une première étape (a1), le composé de formule (I) ou le mélange de composés de formule (I) en introduisant un sucre réducteur et un polyol de formule (A1) ou (B1) tels que par exemple l'érythritol, le xylitol, le glycérol, le diglycérol, le triglycérol ou le sorbitol, dans un réacteur, selon un rapport stoechiométrique maîtrisé, et à soumettre ce mélange à une réaction d'acétalisation dans des conditions de température et de vide partiel prédéterminées en présence d'un système catalytique acide. Les composants de ce système catalytique acide seront généralement choisis parmi les acides sulfurique, chlorhydrique, phosphorique, nitrique, hypophosphoreux, méthane-sulfonique, para- toluène sulfonique, trifluoro-méthane sulfonique et les résines échangeuses d'ions acides. Habituellement, la réaction d'acétalisation sera réalisée à une température de 70 à 130°C, sous un vide de 300 à 20 mbars. Lors d'une deuxième étape (b1), un composé de formule (II) ou un mélange de composés de formule (II) est mélangé avec le produit de la réaction obtenu lors de l'étape (a1), par l'intermédiaire d'un système d'agitation permettant d'atteindre un concentré homogène.

**EP 2 001 437 B1**

Si nécessaire, une troisième étape (c1) consiste à introduire un solvant topiquement acceptable tel que défini précédemment au mélange obtenu lors de l'étape (b1), et à poursuivre l'agitation jusqu'à l'obtention d'un concentré homogène.

Une troisième voie de synthèse consiste à soumettre le polyol de formule (A1) ou (B1) à une déshydratation, en présence d'un système catalytique acide, à une température comprise entre 70°C et 130°C, sous vide partiel, avec élimination concomitante de l'eau formée lors du réarrangement intra-moléculaire subi par le polyol lors d'une première étape (a2); puis à acétaliser le polyol déshydraté ainsi obtenu par dispersion d'un sucre réducteur dans le milieu réactionnel et par maintien de celui-ci à une température comprise entre 80°C et 130°C, sous vide partiel, lors d'une deuxième étape (b2).

Le système catalytique acide utilisé dans cette troisième voie de synthèse peut être identique à celui évoqué pour la deuxième voie.

Si nécessaire, une troisième étape (c2) consiste à introduire un solvant topiquement acceptable tel que défini précédemment au mélange obtenu lors de l'étape (b2), et à poursuivre l'agitation jusqu'à l'obtention d'un concentré homogène.

Une quatrième voie de synthèse par trans-acétalisation consiste à : préparer du butylglucoside par réaction entre le butanol et le glucose en présence d'un système catalytique acide, à une température comprise entre 90°C et 105°C, sous vide partiel, avec élimination concomitante de l'eau formée lors de la réaction lors d'une première étape (a3), le système catalytique acide utilisé pouvant être identique à celui évoqué pour les voies de synthèse précédentes ; ajouter un polyol de formule (A1) ou (B1) au milieu réactionnel ainsi obtenu, avec évacuation par distillation sous vide du butanol résiduel, du butanol formé au cours de la réaction de trans-acétalisation, et de l'eau éventuellement générée lors du réarrangement intra-moléculaire dudit polyol lors d'une deuxième étape (b3) ; et si nécessaire, une troisième étape (c3) consiste à introduire un solvant topiquement acceptable tel que défini précédemment au mélange obtenu lors de l'étape (b3), et à poursuivre l'agitation jusqu'à l'obtention d'un concentré homogène.

Selon un troisième aspect, l'invention a pour objet l'utilisation d'un composé de formule (I), d'un mélange de composés de formule (I) ou du concentré tel que défini précédemment, pour améliorer la tolérance oculaire d'une composition cosmétique, dermocosmétique, dermopharmaceutique ou pharmaceutique à usage topique.

Les composés de formule (I), les mélanges de composés de formule (I) ainsi que les concentrés tel que définis précédemment, peuvent être incorporés dans tout type de composition cosmétique, dermocosmétique, dermopharmaceutique ou pharmaceutique destinée à un usage topique, ou bien encore dans tout type de support destiné à être mis en contact avec la peau (papier, lingette, textile, dispositif transdermique, etc.). Les compositions cosmétiques, à usage topique dans lesquelles est incorporée une quantité efficace de composés de formule (I) ou de mélanges de composés de formule (I), et qui contiennent éventuellement un ou plusieurs tensioactifs moussants et/ou détergents, ou bien les concentrés précédemment définis, peuvent être appliquées indifféremment sur la peau, sur les cheveux, sur le cuir chevelu et sur les muqueuses, et se présentent notamment sous la forme d'une solution, d'une émulsion ou d'une micro-émulsion du type eau dans huile (E/H) ou huile-dans-eau (H/E), d'une émulsion multiple de type eau-dans-huile-dans-eau (E/H/E) ou huile-dans-eau-dans-huile (H/E/H), d'un gel, d'un savon, d'un baume, d'une hydro-dispersion, d'un bâton solide, d'une pommade, d'une crème, d'une mousse, d'un aérosol ou encore sous forme anhydre comme une poudre. Ces compositions peuvent être utilisées comme laits nettoyants ou démaquillants, comme lotions nettoyantes ou démaquillantes, comme gels moussants pour le visage ou pour le corps, comme shampooing ou après-shampooing, comme bain moussant.

De façon générale ces compositions comportent, en plus des tensioactifs moussants et/ou détergents qui peuvent y être éventuellement présents, des excipients et ou des principes actifs habituellement mis en oeuvre dans le domaine des formulations à usage topique, en particulier cosmétiques, dermocosmétiques, pharmaceutiques ou dermo-pharmaceutiques, les épaississants, les gélifiants, les stabilisants, les composés filmogènes, les solvants et co-solvants, les agents hydrotropes, les agents plastifiants, les matières grasses, les huiles, les agents émulsionnants et co-émulsionnants, les agents opacifiants, les agents nacrants, les agents surgraissants, les séquestrants, les agents chélatants, les antioxydants, les parfums, les conservateurs, les agents conditionneurs, les agents blanchissants destinés à la décoloration des poils et de la peau, les principes actifs destinés à apporter une action traitante vis à vis de la peau ou des cheveux, les filtres solaires, les charges minérales ou les pigments, les particules procurant un effet visuel ou destinées à l'encapsulation d'actifs, les particules exfoliantes, les agents de texture, les azurants optiques, les repellents pour les insectes.

Comme exemples de polymères épaississants et/ou gélifiants éventuellement présents dans la composition pour laquelle on met en oeuvre le procédé objet de la présente invention, on peut citer :

- les homopolymères ou copolymères de l'acide acrylique ou de dérivés de l'acide acrylique, les homopolymères ou copolymères de l'acrylamide, les homopolymères ou copolymères de dérivés de l'acrylamide, les homopolymères ou copolymères de l'acide acrylamidométhyl propanesulfonique, de monomère vinylique et/ou de chlorure de triméthylaminoéthylacrylate commercialisés sous les noms CARBOPOL™, ULTREZ™ 10, PEMULEN™ TR1, PEMULEN™ TR2, SIMULGEL™ EG, SIMULGEL™ EPG LUVIGEL™ EM, SALCARE™ SC91, SALCARE™ SC92, SALCARE™ SC95, SALCARE™ SC96, FLOCARE™ ET100, FLOCARE™ ET58, HISPAGEL™, SEPIGEL™ 305, SEPIGEL™ 501, SEPIGEL™ 502, SIMULGEL™ NS, SIMULGEL™ 800, SIMULGEL™ A, SEPIPLUS™ 250, SEPIPLUS™ 265, SEPIPLUS™ 400, SEPINOV™ EMT 10, NOVEMER™ EC1, ARISTOFLEX™ AVC, ARISTOFLEX™ HBM, RAPITIX™ A60, RAPITIX™ A100, COSMEDIA SP et STABILEZE™ 06 ;

- les hydrocolloïdes d'origine végétale ou biosynthétique, par exemple la gomme de xanthane, la gomme de karaya, les carraghénates, les alginates, les galactomannanes ;
- les silicates ; la cellulose et ses dérivés ; l'amidon et ses dérivés hydrophiles ; les polyuréthanes.

Comme exemples de tensioactifs épaississants et/ou gélifiants éventuellement présents dans la composition pour laquelle on met en oeuvre le procédé objet de la présente invention, on peut citer :

- les esters gras d'alkylpolyglycosides éventuellement alkoxylés, et tout particulièrement les esters de méthylpolyglucoside éthoxylés tels que le PEG 120 méthyl glucose trioléate et le PEG 120 méthyl glucose dioléate commercialisés respectivement sous les appellations GLUCAMATE™ LT et GLUMATE™ DOE120 ;
- les esters gras alkoxylés tels que le PEG 150 pentaérythrytyl tétrastéarate commercialisé sous l'appellation CROTHIX™ DS53, le PEG 55 propylene glycol oléate commercialisé sous l'appellation ANTIL™ 141 ;
- les carbamates de polyalkylène glycols à chaînes grasses tels que le PPG 14 laureth isophoryl dicarbamate commercialisé sous l'appellation ELFACOS™ T211, le PPG 14 palmeth 60 hexyl dicarbamate commercialisé sous l'appellation ELFACOS™ GT2125.

Comme exemples d'émulsionnants éventuellement présents dans la composition pour laquelle on met en oeuvre le procédé objet de la présente invention, on peut citer :

- les acides gras, les acides gras éthoxylés, les esters d'acide gras et de sorbitol, les esters d'acides gras éthoxylés, les polysorbates, les esters de polyglycérol, les alcools gras éthoxylés, les esters de sucrose, les alkylpolyglycosides, les alcools gras sulfatés et phosphatés ou les mélanges d'alkylpolyglycosides et d'alcools gras, comme ceus décrits dans les demandes de brevet français 2 668 080, 2 734 496, 2 756 195, 2 762 317, 2 784 680, 2 784 904, 2 791 565, 2 790 977, 2 807 435, 2 804 432, 2 830 774, 2 830 445, les associations de tensioactifs émulsionnants choisis parmi les alkylpolyglycosides, les associations d'alkylpolyglycosides et d'alcools gras, les esters de polyglycérols ou de polyglycols ou de polyols tels que les polyhydroxystéarates de polyglycols ou de polyglycérols mis en oeuvre dans les demandes de brevets français 2 852 257, 2 858 554, 2 820 316 et 2 852 258.

Comme exemples d'agents opacifiants et/ou nacrants éventuellement présents dans la composition pour laquelle on met en oeuvre le procédé objet de la présente invention,, on peut citer les palmitates ou les stéarates ou les hydroxystéarates de sodium ou de magnésium, les monostéarates ou distéarates d'éthylène ou de polyéthylène glycol, les alcools gras, les homopolymères et copolymères de styrène tels que le styrène acrylate copolymère commercialisé sous l'appellation MONTOPOL™ OP1 par la société SEPPIC.

Comme exemples d'huiles éventuellement présents dans la composition pour laquelle on met en oeuvre le procédé objet de la présente invention, on peut citer :

- les huiles minérales telles que l'huile de paraffine, l'huile de vaseline, les isoparaffmes ou les huiles blanches minérales ;
- les huiles d'origine animale, telles que le squalène ou le squalane ;
- les huiles végétales, telles que le phytosqualane, l'huile d'amandes douces, l'huile de coprah, l'huile de ricin, l'huile de jojoba, l'huile d'olive, l'huile de colza, l'huile d'arachide, l'huile de tournesol, l'huile de germes de blé, l'huile de germes de maïs, l'huile de soja, l'huile de coton, l'huile de luzerne, l'huile de pavot, l'huile de potiron, l'huile d'onagre, l'huile de millet, l'huile d'orge, l'huile de seigle, l'huile de carthame, l'huile de bancoulier, l'huile de passiflore, l'huile de noisette, l'huile de palme, le beurre de karité, l'huile de noyau d'abricot, l'huile de calophyllum, l'huile de sysymbrium, l'huile d'avocat, l'huile de calendula, les huiles issues de fleurs ou de légumes ;
- les huiles végétales éthoxylées ;
- les huiles synthétiques comme les esters d'acides gras tels que le myristate de butyle, le myristate de propyle, le myristate de cétyle, le palmitate d'isopropyle, le stéarate de butyle, le stéarate d'hexadécyle, le stéarate d'isopropyle, le stéarate d'octyle, le stéarate d'isocétyle, l'oléate dodécyle, le laurate d'hexyle, le dicaprylate de propylèneglycol, les esters dérivés d'acide lanolique, tels que le lanolate d'isopropyle, le lanolate d'isocétyle, les monoglycérides, diglycérides et triglycérides d'acides gras comme le triheptanoate de glycérol, les alkylbenzoates, les huiles hydrogénées, les polyalphaoléfines, les polyoléfines comme le polyisobutène, les isoalcanes de synthèse comme l'isohexadécane, l'isododécane, les huiles perfluorées et

EP 2 001 437 B1

- les huiles de silicone comme les diméthylpolysiloxanes, les méthylphényl - polysiloxanes, les silicones modifiées par des aminés, les silicones modifiés par des acides gras, les silicones modifiés par des alcools, les silicones modifiés par des alcools et des acides gras, des silicones modifiés par des groupements polyéther, des silicones époxy modifiés, des silicones modifiées par des groupements fluorés, des silicones cycliques et des silicones modifiées par des groupements alkyles.

Comme autre matière grasse éventuellement présente dans la composition pour laquelle on met en oeuvre le procédé objet de la présente invention, on peut citer les alcools gras ou les acides gras ; les cires telles que la cire d'abeille, la cire de carnauba, la cire de candelilla, la cire d'ouricoury, la cire du Japon, la cire de fibre de liège, la cire de canne à sucre, les cires de paraffines, les cires de lignite, les cires microcristallines, la cire de lanoline ; l'ozokérite ; la cire de polyéthylène , les cires de silicone ; les cires végétales ; les alcools gras et les acides gras solides à température ambiante ; les glycérides solides à température ambiante.

Comme exemples de principes actifs éventuellement présents dans la composition pour laquelle on met en oeuvre le procédé objet de la présente invention, on peut citer les composés ayant une action éclaircissante ou dépigmentante, une action hydratante, une action tenseur, une action apaisante ou relaxante, une action anti inflammatoire, une action amincissante, une action lipolytique, une action drainante, une action détoxifiante, une action une action énergisante, décontractante, une action stimulante, une action émoliente, une action neuromodulatrice, une action protectrice, une action purifiante, séborégulatrice, antichute, une action anti-age, une action raffermissante, restructurante, antiradicalaire, ou antioxydante ; de tels principes actifs sont par exemple le SEPIWHITE™MSH, l'arbutine, l'acide kojique, l'hydroquinone, l'acide ellagique, la vitamine C et ses dérivés, le Stay C, le magnésium ascorbyl phosphate et ses dérivés, l'acorbyl glucoside, l'acide phytique, les acides de fruits, le rucinol ou resorcinol, l'acide azélaïque, l'acide lipoïque, le VEGEWHITE™, la GATILUNE™, le SYNERLIGHT™, le BIOWHITE™, le PHYTOLIGHT™, le DERMALIGHT™, la CLARISKIN™, le MELASLOW™, le DERMAWHITE™, l'ETHIOLINE, le MELAREST™, le GIGAWHITE™, l'ALBATINE™, le LUMISKIN™, les extraits de polyphénols, les extraits de raisin, les extraits de pin, les extraits de vin, les extraits d'olives, les extraits de mare, les protéines N-acylées, les peptides N-acylés comme par exemple le MATRIXIL™, les acides aminés N - acylés, les hydrolysâts partiels de protéines N-acylés, les acides aminés, les peptides, les hydrolysâts totaux de protéines, les polyols (par exemple, la glycérine ou le butylène glycol), les dérivés de lait, ou les différents composants entrant dans la composition du NMF (natural moisturizing factor) par exemples l'urée, l'acide pyrrolidone carboxylique ou les dérivés de cet acide, les acides aminés, les sels minéraux, les glucosamines, l'acide glycyrrhétinique, l'alpha-bisabolol, les sucres ou les dérivés des sucres, les polysaccharides ou leurs dérivés, les hydroxyacides par exemple l'acide lactique, les vitamines, les dérivés de vitamines, par exemple le Rétinol, la vitamine E et ses dérivés, les oligo-éléments, les extraits de roches ou pierres, les enzymes, les co-enzymes, comme, le Coenzyme Q10, les hormones ou "hormone like" comme par exemple le PHYTO AGE™, les extraits de soja, par exemple la Raffermine™, les extraits de blé par exemple la TENSINE™ ou la GLIADINE™, les extraits végétaux, tels que les extraits végétaux riches en tanins, les extraits végétaux riches en isoflavones ou les extraits végétaux riches en terpènes, les extraits d'algues d'eau douce ou marines, les extraits marins en général comme les coraux, les cires essentielles, les extraits bactériens, les minéraux comme les GIVOBIO™, les dérivés de calcium, de magnésium, de cuivre, de cobalt, de zinc, de lithium, ou de manganèse, les sels d'argent ou d'or, les lipides en général, les lipides tels que les céramides ou les phospholipides, les actifs ayant une action amincissante ou lipolytique comme la caféine ou ses dérivés, le calcium et ses dérivés, le LIPASLIM™, les actifs améliorant la microcirculation cutanée, par exemple les veinotoniques, les actifs drainants, les actifs à visée décongestionnante tels que le ginko biloba, le lierre, le marron d'inde, le bambou, le ruscus, le petit houx, la centalla asiatica, le fucus le romarins, le saule, les actifs ayant une activité antimicrobienne ou une action purifiantes vis à vis des peaux grasses, par exemple, le LIPACIDE™ C8G, le LIPACIDE™ UG, le SEPICONTROL™ A5, les dérivés de cuivre ou de zinc, l'OCTOPIROX™ ou le SENSIVA™ SC50, les actifs ayant une propriété énergisante ou stimulante comme le SEPITONIC™ M3 ou le Physiogényl™ le panthénol et ses dérivés comme le SEPICAP™ MP, les actifs anti-age comme le SEPILIFT™ DPHP, le LIPACIDE™ PVB, le SEPIVINOL™, le SEPIVITAL™, le MANOLIVA™, le PHYTO-AGE™, les actifs hydratants comme le SEPICALM™ S, le SEPICALM™ VG et le LIPACIDE™ DPHP, les actifs anti-photo vieillissement ", les actifs protecteurs de l'intégrité de la jonction dermo-épidermique, les actifs augmentant la synthèse des composants de la matrice extracellulaire par exemple, le collagène, les élastines, les glycosaminoglycanes, les actifs agissant favorablement sur la communication cellulaire chimique comme les cytokines ou physiques comme les intégrines, les actifs créant une sensation de « chauffe » sur la peau comme les activateurs de la microcirculation cutanée (exemple des nicotinates) ou des produits créant une sensation de « fraîcheur » sur la peau (exemple du menthol et des dérivés).

Comme filtres solaires éventuellement présents dans la composition pour laquelle on met en oeuvre le procédé objet de la présente invention, on peut citer tous ceux figurant dans la directive cosmétique 76/768/CEE modifiée annexe VII.

Les exemples suivants illustrent l'invention, sans toutefois la limiter.

8

**A)- Préparation des composés de formule (I) ou de mélanges de composés de formule (I), et des concentrés mis en oeuvre dans l'invention**

EXEMPLE 1 : préparation du concentré 1 de xylitylglucosides /Lauryléther(2,2 OE) sulfate de sodium/Xylitol.

a) - On introduit 703,0 g de xylitol dans un réacteur en verre à double enveloppe, dans laquelle circule un fluide caloporteur, et muni d'une agitation efficace.

Le xylitol est fondu à une température de 135°C, et la pâte visqueuse ainsi obtenue est refroidie à 115°C.

Le glucose est alors ajouté progressivement au milieu réactionnel pour permettre sa dispersion homogène.

On ajoute au mélange ainsi obtenu un système catalytique acide constitué de 1,29 g d'acide sulfurique à 96 %.

Le milieu réactionnel est placé sous un vide partiel de 90 mbars à 45 mbars, et maintenu à une température de 100°C-105°C pendant une durée de 4 h 30 avec évacuation de l'eau formée au moyen d'un montage de distillation.

Le milieu réactionnel est ensuite refroidi à 95°C-100°C et neutralisé par ajout de 5 g de soude à 30 %, pour amener le pH d'une solution à 1 % de ce mélange à une valeur de 5,0.

Les caractéristiques du mélange intermédiaire ainsi obtenu sont les suivantes :

Aspect (visuel) : cire orange à température ambiante ;
pH solution à 1 % : 5,0 ;
xylitol résiduel : 55,8 % ;
glucose résiduel : < 1 %.
Xylityl glucosides : 37,2%

b) - 15,86 g du mélange intermédiaire précédemment obtenu et 382,15 g de Lauryléther(2,2 OE) sulfate de sodium à 28 % dans l'eau sont mélangés à 50°C dans un réacteur en verre à double enveloppe, dans laquelle circule un fluide caloporteur, et muni d'une agitation efficace. Après obtention d'un mélange homogène, la composition massique du concentré 1 obtenu est la suivante pour 100% de sa matière sèche :

Lauryléther(2,2 OE) sulfate de sodium = 70%
Xylityl glucosides = 12%
Xylitol = 18%

A partir du même mélange intermédiaire obtenu au paragraphe a) et en utilisant des proportions massiques adaptées de Lauryléther(2,2 OE) sulfate de sodium à 28 % dans l'eau, les concentrés 1a et 1b sont obtenus de façon à contenir respectivement 60% massique et 50% massique de matière sèche de Lauryléther(2,2 OE) sulfate de sodium. Les compositions massiques de ces concentrés sont décrites dans le Tableau 1 ci-dessous.

EXEMPLE 2 : préparation du concentré 2 de digycérylglucosides /Lauryléther(2,2 OE) sulfate de sodium/Diglycérol.

a) - On introduit 645,0 g de diglycérol dans un réacteur en verre à double enveloppe, dans laquelle circule un fluide caloporteur, et muni d'une agitation efficace. Le diglycérol est amené à une température d'environ 100°C.

139,9 g de glucose sont alors ajoutés progressivement au milieu réactionnel pour permettre sa dispersion homogène.

On ajoute au mélange ainsi obtenu un système catalytique acide constitué de 0,97 g d'acide sulfurique à 98 %.

Le milieu réactionnel est placé sous un vide partiel à 30 mbars, et maintenu à une température de 100°C-105°C pendant une durée de 4 h 00 avec évacuation de l'eau formée au moyen d'un montage de distillation.

Le milieu réactionnel est ensuite refroidi à 95°C-100°C et neutralisé par ajout de soude à 30 %, pour amener le pH d'une solution à 1 % de ce mélange à une valeur d'environ 7,0.

Les caractéristiques du mélange intermédiaire ainsi obtenu sont les suivantes :

Aspect (visuel) : liquide limpide;
pH solution à 1 % : 6,8;
Diglycérol résiduel : 67,2 % ;
glucose résiduel : < 1 %.
Diglycéryl glucosides : 24,7%

b) - 20,0 g du mélange intermédiaire précédemment obtenu et 166,7 g de Lauryléther(2,2 OE) sulfate de sodium à 28 % dans l'eau sont mélangés à 50°C dans un réacteur en verre à double enveloppe, dans laquelle circule un fluide caloporteur, et muni d'une agitation efficace. Après obtention d'un mélange homogène, la composition massique du concentré 2 obtenu est la suivante pour 100% de sa matière sèche :

Lauryléther(2,2 OE) sulfate de sodium = 70%
Diglycéryl glucosides = 8%
Diglycérol = 22%

A partir du même mélange intermédiaire obtenu à l'étape a) et en utilisant des proportions massiques adaptées de Lauryléther(2,2 OE) sulfate de sodium à 28 % dans l'eau, les concentrés 2a et 2b sont obtenus de façon à contenir respectivement 60% massique et 50% massique de matière sèche de Lauryléther(2,2 OE) sulfate de sodium. Les compositions massiques de ces concentrés sont décrites dans le Tableau 1 ci-dessous.

EXEMPLES COMPARATIFS 2 et 3.

Les exemples comparatifs 2 et 3 sont préparées selon le procédé suivant :

- introduction du Lauryléther(2,2 OE) sulfate de sodium à 28 % dans l'eau dans un bêcher à température ambiante.Le milieu est agité par un barreau aimanté couplé à un agitateur magnétique et le polyol (le xylitol pour l'exemple comparatif 2 et le diglycérol pour l'exemple comparatif 3) est introduit progressivement dans des proportions permettant d'atteindre un concentré contenant 50% massique de matière sèche de Lauryléther(2,2 OE) sulfate de sodium et 50% massique de polyol.

EXEMPLE COMPARATIF 4

L'exemple comparatif 4 est préparé selon le procédé suivant :

- introduction du Lauryléther(2,2 OE) sulfate de sodium à 28 % dans l'eau dans un bêcher à température ambiante.
- le milieu est agité par un barreau aimanté couplé à un agitateur magnétique et le PEG-40 cocoate de glycérol est introduit progressivement dans des proportions permettant d'atteindre un concentré contenant 70% massique de matière sèche de Lauryléther(2,2 OE) sulfate de sodium et 30% massique de PEG-40 cocoate de glycérol.

EXEMPLE COMPARATIF 5

L'exemple comparatif 5 est préparé selon le procédé suivant :

- introduction du Lauryléther(2,2 OE) sulfate de sodium à 28 % dans l'eau dans un bêcher à température ambiante.
- le milieu est agité par un barreau aimanté couplé à un agitateur magnétique et PEG-80 laurate de sorbitan est introduit progressivement dans des proportions permettant d'atteindre un concentré contenant 70% massique de matière sèche de Lauryléther(2,4 OE) sulfate de sodium et 30% massique de PEG-80 laurate de sorbitan.

TABLEAU RECAPITULATIF

Le tableau 1 ci-dessous récapitule les compositions massiques en matières sèches des différents concentrés précédemment préparés et destinés à être mis en oeuvre dans un test d'évaluation de la tolérance oculaire.

Tableau 1 : compositions massiques en matière sèche des concentrés préparés pour l'évaluation de la tolérance oculaire.

|  | Lauryléther(2,2 OE) sulfate de sodium | Xylityl glucosides | Diglycéryl glucosides | Xylitol | Diglycérol | PEG-40 cocoate de glycérol | PEG-80 laurate de sorbitan |
|---|---|---|---|---|---|---|---|
| Concentré 1 | 70% | 12% | - | 18% | - | - | - |
| Concentré 1a | 60% | 16% | - | 24% | - | - | - |

(suite)

| | Lauryléther(2,2 OE) sulfate de sodium | Xylityl glucosides | Diglycéryl glucosides | Xylitol | Diglycérol | PEG-40 cocoate de glycérol | PEG-80 laurate de sorbitan |
|---|---|---|---|---|---|---|---|
| Concentré 1b | 50% | 20% | - | 30% | - | - | - |
| Concentré 2 | 70% | - | 8% | - | 22% | - | - |
| Concentré 2a | 60% | - | 11% | - | 29% | - | - |
| Concentré 2b | 50% | - | 14% | - | 36% | - | - |
| Exemple comparatif 1 | 100% | - | - | - | - | - | - |
| Exemple comparatif 2 | 50% | - | - | 50% | - | - | - |
| Exemple comparatif 3 | 50% | - | - | - | 50% | - | - |
| Exemple comparatif 4 | 70% | - | - | - | - | 30% | - |
| Exemple comparatif 5 | 70% | - | - | - | - | - | 30% |

B) - <u>Mise en évidence des propriétés des composés de formule (I) ou de mélange de composés de formule (I), et de concentrés mis en l'oeuvre dans l'invention</u>

<u>1- Choix de la méthode d'évaluation</u>

La mise en évidence des propriétés des composés de formule (I) ou des mélanges de formule (I) et des concentrés précédemment définis faisant l'objet de l'invention peut être réalisée par l'utilisation de plusieurs méthodes d'évaluation in vitro ; celles-ci étant préférées aux méthodes in vivo pour des raisons éthiques et pour leur facilité de mise en oeuvre.

Parmi les méthodes in vitro, on peut distinguer le test RBCA, mesurant les effets des produits sur la membrane cytoplasmique par la détermination de la concentration de demi-hémolyse, dont l'interprétation par la classification de Pape s'effectue par le ratio hémolyse/dénaturation (1992, INVITTOX data bank, protocol n°37, Russel & Burch, 96-98 North Sherwood, Nottingham, UK) et la méthode HET-CAM (voir références ci-dessous).

Ces méthodes in vitro peuvent être considérées comme alternative au test de DRAIZE, tel que décrit par le protocole OCDE 405, pratiqué sur l'animal (lapin).

Les résultats du test HET-CAM présentent une excellente corrélation avec les résultats du test de DRAIZE sur la famille des alkylpolyglycosides ; cependant, la membrane de l'oeuf étant beaucoup plus sensible que la cornée l'oeil, un facteur 10 permet d'obtenir une parfaite correspondance : un même résultat est obtenu pour une dose de test 10 fois moindre en HET-CAM. La méthode in vitro retenue pour mettre en évidence les propriétés de non irritation oculaire dans le cadre de la présente invention est donc la méthode HET-CAM.

<u>2- Principe de la méthode Het Cam retenue</u>

Les propriétés de non irritation oculaire des composés de formule (I) ou des mélanges de formule (I) et des concentrés précédemment définis, relatives à l'invention ont été mises en évidence par un essai sur la membrane chorio - allantoïdienne de l'oeuf de poule dénommé :"test "HETCAM" (en anglais : Hen Egg Test Chorio Allantoidic Membrane).

Le protocole expérimental de cet essai est adapté de la publication LUEPKE (The Hen's egg test: «An alternative toxicity test » Brit J. Dermato. 1986, 115. Suppl.31, 133-135, LUEPKE N.P. & KEMPER F.H. The HET-CAM test: «an alternative to the Draize test ». Food Chem. Toxicol. 1986, 24, n°6/7, 495-496), du protocole INVITTOX n°47 (1992, INVITTOX Data Bank, 34 Stoney Street, Nottingham NG1 1 NB, UK) et de l'annexe IV de l'arrêté publié au Journal Officiel de la république française du 26 décembre 1996, relatif aux méthodes officielles d'analyse des produits de beauté (NOR : FCEC9600217A).

Ce test consiste à apprécier le potentiel d'irritation d'une substance par lecture des modifications observées après application d'une solution de cette substance sur la membrane chorio - allantoïdienne richement vascularisée de l'oeuf de poule de race LEGHORN pré - incubé.

3 - Protocole expérimental

Les produits ont été testés à une concentration de 1% en matière active. Les solutions sont préparées par dilution dans l'eau permutée au maximum vingt-quatre heures à l'avance.

Le pH des solutions d'essai est ajusté à 7 avec du triéthanolamine ou de l'acide lactique si nécessaire.

A réception, les oeufs fertilisés sont pré-incubés en couveuse pendant dix jours à 37,8°C $\pm$ 0,5 °C, avec un taux d'humidité dans la couveuse de 50 % à 60 %.

On découpe la coquille des oeufs sur lesquels sont effectués les essais, autour de la poche d'air et l'on découvre la membrane chorio-allantoïdienne en retirant délicatement la membrane coquillère à l'aide de pince.

On dépose à la surface de la membrane, 0,3 cm$^3$ de la solution à tester.

Vingt secondes après, la membrane est rincée avec 5 cm$^3$ d'eau permutée à 37 °C pendant 10 secondes.

On repère sur un intervalle de temps de cinq minutes, le début :

d'une hyperémie (temps $t_1$),
d'une hémorragie (temps $t_2$)
et de la coagulation (temps $t_3$)

Par définition, si l'un des phénomènes n'est pas apparu au cours de ces cinq minutes (300 secondes), il est noté $t_i$ = 301 secondes.

4- Expression des résultats

Chaque solution est testée sur 4 à 6 oeufs en fonction de la répétitivité des réactions observées. L'indice HetCam I, représentatif du test, est calculé pour chaque oeuf testé de la manière suivante :

$$I = 5 \times (301-t_1) / 300 + 7 \times (301-t_2) / 300 + 9 \times (301-t_3) / 300$$

La moyenne des indices individuels est ensuite calculée ainsi que l'incertitude statistique i :

$$i = t . S / \sqrt{n} \text{ pour un risque de 5\%}$$

Le barème suivant de l'indice I est adopté pour l'interprétation des résultats :

| Indice Hetcam | Classification du composé testé |
|---|---|
| I < 1 | Non irritant |
| 1 $\leq$ I < 5 | Faiblement irritant |
| 5 $\leq$... I < 9 | Modérément irritant |
| 9 $\leq$... I < 12 | Irritant |
| I $\geq$ 12 | Sévèrement irritant |

Compte tenu du facteur de correspondance entre ce test, in vitro, et le vivo, le résultat obtenu en HET-CAM pour une solution à 1%MA est équivalent à celui obtenu pour une solution à 10%MA in vivo.

5- Influence des composés de formule (I) ou des mélanges de composés de formule (I) sur la tolérance oculaire de composés de formule (II).

5.1. Résultats obtenus

Tableau 2 : résultats des tests Het-Cam des concentrés préparés.

|  | Indice Hetcam | Classification du concentré testé |
|---|---|---|
| Concentré 1 | 3,0 | Légèrement irritant |
| Concentré 1a | 0,9 | Non irritant |
| Concentré 1b | 0 | Non irritant |
| Concentré 2 | 3,0 | Légèrement irritant |
| Concentré 2a | 2,4 | Légèrement irritant |
| Concentré 2b | 0,9 | Non irritant |
| Exemple comparatif 1 | 10,7 | Irritant |
| Exemple comparatif 2 | 8,2 | Modérément irritant |
| Exemple comparatif 3 | 9,0 | Irritant |
| Exemple comparatif 4 | 6,0 | Modérément irritant |
| Exemple comparatif 5 | 10,5 | Irritant |

5.2. Analyse des résultats

Les résultats sont jugés satisfaisants et par conséquent le procédé selon l'invention est efficace si le concentré testé montre un indice HETCAM inférieur à 5 et de préférence inférieur ou égal à 3.

Les tensioactifs moussants de formule (II) sont classés « irritants » selon le test Het Cam en vigueur (exemple comparatif 1).

L'association avec des tensioactifs non ioniques éthoxylés à hauteur de 30% massique, connu par l'homme du métier pour permettre de diminuer l'irritation oculaire (exemple comparatif 4 et exemple comparatif 5) ne permet pas d'atteindre le classement « légèrement irritant » minimal nécessaire pour une utilisation dans une composition cosmétique, dermocosmétique, dermopharmaceutique ou pharmaceutique à application cutanée pouvant être en contact avec les yeux de l'utilisateur.

L'association des tensioactifs de formule (II) avec des polyols pouvant constituer un solvant topiquement acceptable selon l'invention ne permet pas d'atteindre le classement « légèrement irritant » minimal nécessaire pour une utilisation dans une composition cosmétique, dermocosmétique, dermopharmaceutique ou pharmaceutique à application cutanée pouvant être en contact avec les yeux de l'utilisateur (exemple comparatif 2 et exemple comparatif 3).

Il faut associer 12 % massique de composés de formule (I) ou de mélange de composés de formule (I) sur chaîne xylityle au Lauryléther(2,2 OE) sulfate de sodium de l'exemple comparatif 1 (concentré 1) pour amener le concentré comprenant les tensioactifs de formule (II) à la classification « légèrement irritant » et 16% massique de composés de formule (I) ou de mélange de composés de formule (I) sur chaîne xylityle au Lauryléther(2,2 OE) sulfate de sodium de l'exemple comparatif 1 (concentré 1a) pour amener le concentré à la classification « non irritant ».

Il faut associer 8 % massique de composés de formule (I) ou de mélange de composés de formule (I) sur chaîne diglycéryle au Lauryléther(2,2 OE) sulfate de sodium de l'exemple comparatif 1 (concentré 2) pour amener le concentré comprenant les tensioactifs de formule (II) à la classification « légèrement irritant » et 14% massique de composés de formule (I) ou de mélange de composés de formule (I) sur chaîne diglycéryle au Lauryléther(2,2 OE) sulfate de sodium de l'exemple comparatif 1 (concentré 2b) pour amener le concentré à la classification « non irritant ».

C)-Formulations

Dans les formules suivantes, les pourcentages sont exprimés en poids de la formulation.

C.1 Fluide démaquillant visage

|  | Formule |
|---|---|
| Concentré 1 | 10,00 % |
| Méthyl paraben | 0,15 % |
| Phenoxyethanol | 0,80 % |
| SEPICALM™ S | 1,00 % |
| Parfum/Fragrance | 0,10 % |
| Eau | qs. 100,00 % |

Mode opératoire : mélanger les différents ingrédients dans l'eau sous agitation magnétique dans l'ordre indiqué, et ajuster le pH aux alentours de 7.

C.2 Shampoing cheveux et corps pour enfants

Formule

| A | Concentré 2 | 15,00 % |
|---|---|---|
|  | PROTEOL™ APL | 5,00 % |
|  | SEPICIDE™ HB | 0,50 % |
|  | Parfum/Fragrance | 0,10 % |
| B | Eau | 20,00 % |
|  | CAPIGEL™ 98 | 3,50 % |
| C | Eau | Q.S. 100,00 % |
|  | SEPICIDE™ CI | 0, 30 % |
|  | Colorant | Q.S |
|  | Soude | Q.S. pH = 7,2 |

Mode opératoire : mélanger le concentré 2 avec le PROTEOL™ APL, et le SEPICIDE™ HB (Phase A). Diluer le CAPIGEL™ 98 dans une partie de l'eau et l'ajouter à la phase A précédemment obtenue (Phase B). Ajouter le reste d'eau à la phase B, puis le SEPICIDE™ CI, le colorant. Ajuster le pH du mélange à 7,2 environ avec de la soude.

C.3 Lingettes démaquillantes pour les yeux

|  |  | Formule |
|---|---|---|
| A | Concentré 1 | 3,00 % |
| B | SEPICIDE™ HB2 | 0,50 % |
| C | SEPICALM™ VG | 0,50 % |
|  | Glycérine | 10,00 % |
|  | Parfum/Fragrance | 0,05 % |
| D | Eau | Q.S. 100,00 % |

Mode opératoire : mélanger les ingrédients de la phase B ainsi que ceux de la phase C dans la phase A jusqu'à obtenir la limpidité de la solution. Ajouter la phase D .

C.4 Gel moussant doux

| | Formule | |
|---|---|---|
| A | Concentré 2 | 8,50 % |
| | PROTEOL™ APL | 3,00 % |
| | EUXYL™ PE9010 | 1,00% |
| | Parfum/Fragrance | 0,10% |
| B Eau | | Q.S. 100,00 % |
| | Acide lactique | Q.S. pH = 6,0 |

Mode opératoire : solubiliser le parfum et le conservateur EUXYL™ PE9010 dans le mélange composé du concentré 2 et du PROTEOL™ APL (phase A). Ajouter l'eau et régler le pH à environ 6,0 avec de l'acide lactique.

C.5 Shampoing à usage fréquent

| | Formule | |
|---|---|---|
| A | Concentré 2 | 12,80 % |
| | PROTEOL™ OAT | 5,00 % |
| | EUXYL™ PE9010 | 1,00% |
| | Parfum/Fragrance | 0,30% |
| | Eau | Q.S. 100,00 % |
| B | MONTALINE™ C40 | 8,50 % |
| | Acide lactique | Q.S. pH = 6,0 |

Mode opératoire : mélanger tous les ingrédients de la phase A et, après homogénéisation, ajouter la MONTALINE™ C40 et ajuster le pH à environ 6,0 à l'aide de l'acide lactique.

C.6 Shampoing ultra-doux pour bébé

| | Formule | |
|---|---|---|
| A | Concentré 2 | 10,00% |
| | AMISOFT™ CS-11 | 4,00% |
| | Parfum/Fragrance | 0,10% |
| | SEPICIDE™ HB | 0,30% |
| | SEPICIDE™ CI | 0,20% |
| | Eau | Q.S. 100,00% |
| B | Eau | 20,00% |
| | CAPIGEL™ 98 | 3,50% |
| | Tromethamine | Q.S. pH = 7,2 |

Mode opératoire : mélanger tous les ingrédients de la phase A dans l'ordre indiqué jusqu'à l'obtention d'une phase A limpide. De façon séparée, ajouter le CAPIGEL™ 98 dans l'eau, puis ajouter cette phase B ainsi préparée dans la phase A et ajuster le pH à 7,2 à l'aide de la trométhamine.

C.7 Lait de toilette pour bébé

| | Formule | |
|---|---|---|
| A | SIMULSOL™ 165 | 2,00% |
| | MONTANOV™ 202 | 1,00% |
| | LANOL™ 99 | 3,00% |
| | Dimethicone | 1,00% |
| | Isohexadecane | 3,00% |
| B Eau | | Q.S. 100,00% |
| C | SEPIPLUS™ 400 | 0,30% |
| D | concentré 2 | 6,35% |
| E | SEPICIDE™ HB | 0,30% |
| | DMDM Hydantoin | 0,20% |
| | Parfum/Fragrance | 0,10% |

Mode opératoire : faire chauffer séparément les phases A et B constituées par mélange des différents constituants. Ajouter la phase C dans la phase grasse chaude et réaliser l'émulsion en versant la phase aqueuse ; homogénéiser quelques minutes sous forte agitation (par l'intermédiaire d'une turbine rotor/stator). Puis ajouter la phase D dans l'émulsion chaude et refroidir l'émulsion sous agitation modérée jusqu'à retour à température ambiante. Ajouter la phase E à 40°C.

C.8 Lotion poudrée nettoyante pour peaux sensibles

| | Formule | |
|---|---|---|
| A | LIPACIDE™ C8G | 0,95% |
| | Méthyl paraben | 0,10% |
| | Ethyl paraben | 0,024% |
| | Propyl paraben | 0,0119% |
| | Butyl paraben | 0,024% |
| | Isobutyl paraben | 0,0119% |
| | Eau | 20,00% |
| | Disodium EDTA | 0,10% |
| | Triethanolamine | 1,38% |
| B | concentré 2 | 1,80% |
| | Parfum/Fragrance | 0,10% |
| C | SEPICALM™ S | 0,28% |
| | Eau | Q.S. 100,00% |
| | Acide lactique | Q.S. pH = 5,2 |
| D | MICROPEARL™ M310 | 5,00% |

Mode opératoire : solubiliser les ingrédients de la phase A dans l'eau à 80°C. Solubiliser séparément le parfum dans le concentré 2 pour préparer la phase B. Ajouter la phase A refroidie sur la phase B, puis introduire le SEPICALM™ S et le complément d'eau. Vérifier le pH final et éventuellement l'ajuster à environ 5,2. Ajouter alors le MICROPEARL™ M310.

Les caractéristiques des produits utilisés dans les exemples précédents, sont les suivantes :

Le SEPICALM™ S est un mélange de N-cocoyl aminoacides, de sarcosine, d'aspartate de potassium et d'aspartate de magnésium tel que décrit dans WO 98/09611 et commercialisé par la société SEPPIC.
Le PROTEOL™ APL est un mélange de N-cocoyl aminoacides sous forme de sels de sodium, obtenus par acylation des acides aminés caractéristiques du jus de pomme et commercialisé par la société SEPPIC.
Le SEPICIDE™ HB, un mélange de phénoxyéthanol, de méthylparaben, d'éthylparaben, de propylparaben et de butylparaben, est un agent conservateur commercialisé par la société SEPPIC.
Le CAPIGEL™ 98 est un copolymère d'acrylates commercialisé par la société SEPPIC.

Le SEPICIDE™ CI, imidazoline urée, est un agent conservateur commercialisé par la société SEPPIC.

Le SEPICIDE™ HB2, un mélange de phénoxyéthanol, de méthylparaben, d'éthylparaben, de propylparaben, de butylparaben et d'isobutylparaben, est un agent conservateur commercialisé par la société SEPPIC.

Le SEPICALM™ VG est un mélange de N-palmitoyl proline sous forme de sel de sodium et d'extrait de fleur de Nymphéa Alba, commercialisé par la société SEPPIC.

L' EUXYL™ PE9010, mélange de phénoxyéthanol et d'Ethyl Hexyl Glycérine, est un agent conservateur commercialisé par la société SEPPIC.

Le PROTEOL™ OAT est un mélange de N-lauryl aminoacides obtenus par hydrolyse totale de protéine d'avoine tel que décrit dans WO 94/26694 et commercialisé par la société SEPPIC.

La MONTALINE™ C40 est un sel de chlorure de Cocamidopropyl betaïnamide de Monoéthanolamine.

L' AMISOFT™ CS-11 est un sel de sodium de N-cocoyl glutamate, commercialisé par la société AJINOMOTO.

Le SIMULSOL™ 165 est un mélange de stéaraate de PEG-100 et de stéarate de glycérol, commercialisé par la société SEPPIC.

Le MONTANOV™ 202 (alcool arachydilique, alcool béhénique et arachidyl glucoside), est une composition auto-émulsionnable telle que celles décrites dans EP 0 977 626, commercialisée par la société SEPPIC.

Le LANOL™ 99 est l'isononoate d'isononyle commercialisé par la société SEPPIC.

Le SEPIPLUS™ 400 est un latex inverse auto - inversible de polyacrylates dans le polyisobutene et comportant du polysorbate 20, tel que décrit dans WO2005/040230 et commercialisé par la société SEPPIC.

Le LIPACIDE™ C8G est du capryloyl glycine commercialisé par la société SEPPIC.

Le MICROPEARL™ M310 est un polymère polyméthylméthacrylate réticulé se présentant sous forme de poudre et utilisé comme modificateur de texture.

**Revendications**

1. Procédé pour améliorer la tolérance oculaire d'une composition à usage topique, **caractérisé en ce que** l'on incorpore dans ladite composition, une quantité efficace d'un composé de formule (I):

$$R_1\text{-O-}(G)_x\text{-H} \qquad\qquad (I)$$

dans laquelle :

x représente un nombre décimal compris entre 1 et 5,
G représente le reste d'un sucre réducteur et
$R_1$ représente un radical monovalent de formule (A) :

$$-CH_2\text{-}(CHOH)_n\text{-}CH_2\text{-OH} \qquad\qquad (A)$$

dans laquelle n est un nombre entier égal à 2, 3 ou 4, ou bien
$R_1$ représente un radical monovalent de formule (B) :

$$-(CH_2\text{-CHOH-}CH_2\text{-O})_m\text{-H} \qquad\qquad (B)$$

dans laquelle m est un nombre entier égal à 1, 2 ou 3,
ou d'un mélange de composés de formules (I).

2. Procédé tel que défini à la revendication 1, **caractérisé en ce que** dans la formule (I), G représente le reste d'un sucre réducteur choisi parmi le glucose, le dextrose, le saccharose, le fructose, l'idose, le gulose, le galactose, le maltose, l'isomaltose, le maltotriose, le lactose, le cellobiose, le mannose, le ribose, le xylose, l'arabinose, le lyxose, l'allose, l'altrose, le dextrane ou le tallose.

3. Procédé tel que défini à la revendication 2 **caractérisé en ce que** dans la formule (I), G représente le reste d'un sucre réducteur choisi parmi le glucose, le xylose et l'arabinose.

4. Procédé tel que défini à l'une des revendications 1 à 3, **caractérisé en ce que** ladite composition à usage topique comprend au moins un agent tensioactif moussant et/ou détergent.

5. Procédé tel que défini à la revendication 4, **caractérisé en ce que** l'agent tensioactif moussant et/ou détergent est un composé de formule (II) :

$$[R_2\text{-O-}(CH_2\text{-}CH_2\text{-O})_p\text{-SO}_3]_r X \qquad (II)$$

dans laquelle :

R$_2$ représente un radical hydrocarboné aliphatique, saturé ou insaturé, linéaire ou ramifié, comportant de 6 à 22 atomes de carbone,
p représente un nombre décimal compris entre 1 et 10, de préférence entre 2 et 4,
r est un nombre entier égal à 1 ou à 2 et
X représente le cation d'un métal alcalin ou d'un métal alcalino-terreux, l'ion ammonium, l'ion hydroxyéthyl ammonium, l'ion tri(hydroxyéthyl) ammonium ou un mélange de composé de formule (II).

6. Procédé tel que défini à l'une quelconque des revendications 4 ou 5, **caractérisé en ce que** le rapport massique, composé de formule (I) sur agent tensioactif moussant et/ou détergent présents dans ladite composition à usage topique, est compris entre 1/10 et 10/1 et plus particulièrement entre 1/10 et 1/1.

**Patentansprüche**

1. Verfahren zur Verbesserung der Augentoleranz einer Zusammensetzung zur topischen Anwendung, **dadurch gekennzeichnet, dass** in die Zusammensetzung eine wirksame Menge einer Verbindung mit der folgenden Formel (I) integriert wird:

$$R_1\text{-O-}(G)_x\text{-H} \qquad (I)$$

wobei:

x eine Dezimalzahl zwischen 1 und 5 darstellt,
G den Rest eines reduzierenden Zuckers darstellt, und
R$_1$ ein monovalentes Radikal mit der folgenden Formel (A) darstellt:

$$-CH_2\text{-}(CHOH)_n\text{-}CH_2\text{-OH} \qquad (A)$$

wobei n eine ganze Zahl gleich 2, 3 oder 4 ist, oder
R$_1$ ein monovalentes Radikal mit der folgenden Formel (B) darstellt:

$$-(CH_2\text{-CHOH-}CH_2\text{-O})_m\text{-H} \qquad (B)$$

wobei m eine ganze Zahl gleich 1, 2 oder 3 ist,
oder einer Mischung von Verbindungen mit der Formel (I).

2. Verfahren nach Anspruch 1, **dadurch gekennzeichnet, dass** in der Formel (I), G den Rest eines reduzierenden Zuckers darstellt, ausgewählt aus der Glukose, der Dextrose, der Saccharose, der Fruktose, der Idose, der Gulose, der Galaktose, der Maltose, der Isomaltose, der Maltotriose, der Laktose, der Cellobiose, der Mannose, der Ribose, der Xylose, der Arabinose, der Lyxose, der Allose, der Altrose, des Dextrans oder der Tallose.

3. Verfahren nach Anspruch 2, **dadurch gekennzeichnet, dass** in der Formel (I), G den Rest eines reduzierenden Zuckers darstellt, ausgewählt aus der Glukose, der Xylose und der Arabinose.

4. Verfahren nach einem der Ansprüche 1 bis 3, **dadurch gekennzeichnet, dass** die Zusammensetzung zur topischen Anwendung mindestens ein schaumbildendes oberflächenaktives Mittel und/oder Waschmittel umfasst.

5. Verfahren nach Anspruch 4, **dadurch gekennzeichnet, dass** das schaumbildende oberflächenaktive Mittel und/oder Waschmittel eine Verbindung mit der folgenden Formel (II) ist:

$$[R_2\text{-O-}(CH_2\text{-}CH_2\text{-O})_p\text{-SO}_3]_r X \qquad (II)$$

wobei:

R$_2$ ein aliphatisches, gesättigtes oder ungesättigtes, lineares oder verzweigtes Kohlenwasserstoffradikal darstellt, umfassend von 6 bis 22 Kohlenstoffatomen,
p eine Dezimalzahl zwischen 1 und 10, vorzugsweise zwischen 2 und 4 darstellt,
r eine ganze Zahl gleich 1 oder 2 ist, und
X das Kation eines Alkalimetalls oder eines Erdalkalimetalls, das Ammoniumion, das Hydroxyethylammoniumion, das Tri(hydroxyethyl)ammoniumion oder eine Mischung der Verbindung mit der Formel (II) darstellt.

6. Verfahren nach einem der Ansprüche 4 oder 5, **dadurch gekennzeichnet, dass** das Massenverhältnis der Verbindung mit der Formel (I) zum schaumbildenden oberflächenaktiven Mittel und/oder Waschmittel, vorhanden in der Zusammensetzung zur topischen Anwendung, zwischen 1/10 und 10/1 und insbesondere zwischen 1/10 und 1/1 liegt.

**Claims**

1. Method for improving the ocular tolerance of a composition for topical use, **characterised in that** an effective quantity of a compound with formula (I) is incorporated in said composition:

$$R_1\text{-}O\text{-}(G)_x\text{-}H \qquad (I)$$

wherein:

x represents a decimal number between 1 and 5,
G represents the residue of a reducing sugar,
R$_1$ represents a monovalent radical with formula (A)

$$-CH_2\text{-}(CHOH)_n\text{-}CH_2\text{-}OH \qquad (A)$$

wherein n is an integer number equal to 2, 3 or 4, or
R$_1$ represents a monovalent radical with formula (B)

$$-(CH_2\text{-}CHOH\text{-}CH_2\text{-}O)_m\text{-}H \qquad (B)$$

wherein m is an integer number equal to 1, 2, 3,
or a mix of compounds with formula (I)

2. Method as defined in claim 1, **characterised in that** in formula (I), G represents the represents the residue of a reducing sugar chosen from among glucose, dextrose, saccharose, fructose, idose, gulose, galactose, maltose, isomaltose, lactose, cellobiose, mannose, ribose, xylose, arabinose, lyxose, allose, altrose, dextrane and tallose.

3. Method as defined in claim 2, **characterised in that** in formula (I), G represents the represents the residue of a reducing sugar chosen from among glucose, xylose and arabinose.

4. Method as defined in one of claims 1 to 3, **characterised in that** said composition for topical use comprises at least one foaming surfactant and/or detergent.

5. Method as defined in claim 4, **characterised in that** the foaming surfactant and/or detergent is a compound with formula (II):

$$[R_2\text{-}O\text{-}(CH_2\text{-}CH_2\text{-}O)_p\text{-}SO_{3r}]X \qquad (II)$$

wherein:

R$_2$ represents a saturated or unsaturated, linear or branched aliphatic hydrocarbon radical comprising 6 to 22 carbon atoms,
p represents a decimal number between 1 and 10, and preferably between 2 and 4,

r is an integer number equal to 1 or 2 and

X represents the cation of an alkaline metal or an alkali-earth metal, the ammonium ion, the hydroxyethyl ammonium ion, the (tri)hydroxyethyl ammonium ion, or a mix of compounds with formula (II)

6. Method as defined in either of claims 4 and 5, **characterised in that** the mass ratio of the compound with formula (I) to the surfactant and/or detergent present in said composition for topical use is between 1/10 and 10/1, and more particularly between 1/10 and 1/1.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 03094864 A
- US 20020123438 A1
- FR 2668080
- FR 2734496
- FR 2756195
- FR 2762317
- FR 2784680
- FR 2784904
- FR 2791565
- FR 2790977
- FR 2807435
- FR 2804432
- FR 2830774
- FR 2830445
- FR 2852257
- FR 2858554
- FR 2820316
- FR 2852258
- WO 9809611 A
- WO 9426694 A
- EP 0977626 A
- WO 2005040230 A

**Littérature non-brevet citée dans la description**

- **DANIEL VOET ; JUDITH G. VOET.** Biochemistry. John Wyley & Sons, 1990, 250
- An alternative toxicity test. *Brit J. Dermato,* 1986, vol. 115 (31), 133-135
- **LUEPKE N.P. ; KEMPER F.H.** The HET-CAM test: «an alternative to the Draize test ». *Food Chem. Toxicol.,* 1986, vol. 24 (6/7), 495-496